# EUROPEAN PATENT APPLICATION

(11) **EP 1 752 163 A1**
(43) Date of publication of application: **14.02.2007**
(21) Application number: 05749550.9
(22) Date of filing: 13.05.2005
(51) Int. Cl.: A61K 39/395

(54) **AGENT FOR ACTIVELY RESTORING STRUCTURE AND FUNCTIONS OF DAMAGED TISSUES AND ORGANS**

(30) Priority: 14.05.2004 RU 2004115385
(71) Applicant: Bizyaev, Alexey Vyacheslavovich, Moskovskaya obl. 143300 (RU)
(72) Inventor: KRAVETS, Vasily Nikolaevich, St.Petersburg, 196240 (RU); RUGAL, Victor Ivanovich, St.Petersburg, 192281 (RU); GONCHARE, Vladimir Alexandrovich, St.Petersburg 194156 (RU)
(74) Representative: Karlsson, Leif Karl Gunnar
(86) International application number: PCT/RU2005/000277
(87) International publication number: WO 2005/110472

(57) **Abstract**

The inventive agent comprises polyclonal IgG class immunoglobulins whose molecular mass is equal to 150 kD and which are produced in the form of a humoral immune response to a mesenchymal cell antigen CD34 (-) complex.

## Description

### Pertinent art.

The present invention refers to medicine, namely to the medical agents which contain antibodies and antigens. It may be used for biological activity stimulation of elementary mesenchymal cells while treatment of diseases and traumas that require regeneration (recuperation) of injured tissues and organs.

The organism capability to regenerate injured cellular structures and tissues, for example, in case of myocardial infraction, cerebral hemorrhage, bones and vessels pathologies is determined with cells capable to transform into various tissular formations - haematogenous, boned, muscular, sebaceous, nervous and others [Korbling M. et al. A New England J. of Medicine, 2003, v 349, p 570 - 582]. In the grown-ups these cells being in normal are involved in the physiological regeneration of moribund cells and represent latent cells of the long-lasting mesenchymal tissue. The mesenchymal cells do not have CD34(+), CD44(+), CD45(+) antigens and MNS complex antigens [Yiang Y et al. Nature, 2002, v 418, p 41 - 49]. They are localized in periosteal, endosteal and perivascular organism areas. In the same areas somatic stem cells are located. When pathology, a set of adverse factors emersed in the area of injured tissues distorts or makes impossible a signal coming from peripheries to elementary cells to make them more active and provide the injured cells regeneration. The regulatory factors are required for each cell line.

### Antecedent technical level.

It is known to use allogenous or autologous stem haematogenous cells as a regulatory factor while treatment of various diseases [Orlic D. et al. Nature, 2001, v 410, p 701 - 705; Alison M. R. et al. Nature, 2000, v 406, p 257]. The stem cells may be derived from bone marrow, umbilical or peripheral blood and embryonal tissues. The stem cells transplantation effect is dose-dependent: minimum effective dose for the recipient up to 10 kg is equal to 5×10⁸ cells, for the recipient more than 50 kg is equal to 25×10⁸ cells [Romanenko N. A. Storage and preservation of umbilical blood nucleated cells. Dissertation abstract of the medical science candidate, Saint-Petersburg, 2000, 24p.]. The process of stem cells accumulation for transplantation is labour-consuming, expensive and requires serious arrangements concerning an immunohaematologic selection of donor-recipient pairs, carrying out of immunodepression, preventive measures against infectious complications etc. A dose-dependent action of stem cells transplantation results in necessary accumulation of a great number of stem cells which is impossible in some cases, or brings to application of artificial stimulators of their activity [Blan H. M. et al Cell, 2001, v 105, p 829 - 841]. The artificial stimulators of the stem cells activity normally cause adverse reactions and patients complications.

It is also known an antireticular cytotoxic serum by A.A. Bogomolets used in the test as a stimulator for cultivation of certain cells [Myagkaya I.P. Antireticular cytotoxic serum by Bogomolets A. A. as a agent for pathogenetic therapy of various diseases. Physiopathology, 1981, vol 2, p 39-46]. The pointed serum is derived in result of immunization of an animal (for example, horse) with a mixture of a human bone marrow tissues, spleen cells and intestine lymphatic formations without their phenotypical identification. Blood corpuscles were separated out of the immunized animal blood and the serum containing immunoglobulins that appeared as a humoral immune response was being used as a serum.

Disadvantageous features of the antireticular cytotoxic serum by A.A Bogomolets consist in its low selectiveness in activation of the long-lasting mesenchyma considering that bone marrow tissues were used for the immunization of horses entirely without selecting areas enriched with mesenchymal cells which makes unpredictable their entry into antigen structure. The use of spleen cells and intestine lymphatic formations in antigenic material makes the immunological potency of the immunized organism produce immunoglobulins without manifesting activity to the long-lasting mesenchyma cells that reduces a serum selectiveness as well. Besides, the serum contains immunologically inactive ballast proteins but sensitizing the organism of a patient and capable to cause allergic reactions.

### Disclosure of invention.

At the heart of the invention there is an assigned task to enhance the selectiveness of the agent in activation of the long-lasting mesenchyma cells.

In accordance with the invention there is an agent offered to recuperate structures and functions of injured tissues and organs which contains IgG polyclonal immunoglobulins of average molecular weight 150 kD considered to be a humoral immune response to the CD34(-) antigens complex of the mesenchymal cells.

The claimed agent to recuperate structures and functions of injured tissues and organs may be obtained as xenogenous humoral immune response to the CD34(-) antigens complex of the mesenchymal cells.

The IgG-class and IgG1-IgG4-underclass immunoglobulins are of identical constant regions with constant sequence of amino acids and diverse variable regions; a distinction in variable regions brings to distinction of the main effector characteristics. Produced as a humoral response to the CD34(-) mesenchymal cells, they stimulate a biological activity of elementary mesenchymal cells (long-lasting mesenchyma cells) that results in injured tissues and organs regeneration, Thus, the said agent has broad perspectives to be used in clinical practice.

### The claimed agent may be obtained the following way.

To obtain the antigen, a trepanobioptate of donor bone marrow containing endosteal and periosteal mesenchymal cells was washed away of the bone marrow parenchyma of blood corpuscles with a physiological solution, mechanically masticated to homogeneous state and weighed in the physiological solution. The homogeneous suspended material was centrifugated at a speed of 1500 r/min. A supernatant fluid was being frozen trice and defrosted after which masticated again and centrifugated at a speed of 3000 r/min. A protein content in the supernatant fluid was appreciated with a biuretic method and brought to a concentration of 2,2 mg/ml by diluting with the physiological solution.

The antigen obtained this way was injected into an animal 2,2 mg/ml counting on 1 kg of an immunized object. As the immunized object may be taken any biological object capable to give humoral immune response to foreign protein introduction, for example, domestic horned cattle. The first antigen injection was carried out in an entire Freund's adjuvant or another immune response stimulator. Next injections were being held at 2-7 days intervals so that the whole immunization duration take 20-50 days. The immunized object blood is taken into germ-proof utensils without preservatives and anticoagulants. The blood sampling rate of an animal does not exceed 10 ml blood per 1 kg body weight.

After the blood corpuscles sedimentation and clot formation, the supernatant fluid (serum) was put into another germ-proof bottle. Immunoglobulin fractions were separated out of the supernatant fluid by desalting, deposits were separated, dissolved in distilled water and divided into fractions chromatographically. The fraction corresponding to IgG-class was washed away with a special column effluent and cleaned up with a dialysis through the permselective membrane. A purified solution was sterilized by filtration, divided into doses and desiccated in vacuum or inert gas ambient until residual moisture 2-5%. The agent was being kept at a temperature of 4°C in dark place not more than 24 months.

Mesenchymal tissues of periosteal, endosteal and interstitial areas of bone marrow may be used as a source of antigen material - donor CD34(-) human mesenchymal cells. The bone marrow trepanobioptates taken from healthful donors ought to be processed as stated above. The obtained antigen containing CD34(-) mesenchymal cells may be used immediately or kept frozen until application.

### Embodiment of invention.

Hereafter, the present invention is illustrated by examples of preparation and application of claimed agent in vitro and in vivo.

### Example.

A 25 kg disease-free goat was immunized. Four points of its body were injected per 55 mg antigen emulsified in the entire Freund's adjuvant.

After 14 days the same amount of antigen was injected 6 times parenterally without adjuvant at 2-4 days intervals. Accumulated antigen dose was 155 mg × 7 = 3,85 mg.

Before each injection beginning with the fourth, the antibodies (immunoglobulins) titer for mesenchymal cells was being checked. Before the fifth injection, the antibodies titer was 1:512, before the sixth injection - 1:1024, before the blood exfusion - 1:2048. In 7 days after the last antigen injection, 250 ml blood without preservatives was taken from the goat jugular vein.

The serum was separated out of the clot and blood corpuscles by centrifuging at a speed of 3000 r/min during 30 min. Out of the serum was sedimentated a poliglobulin fraction by adding a saturated solution of ammonia sulphate. The supernatant fluid was separated by centrifuging, the deposit was dissolved in distilled water, protein concentration constituted 5 mg/ml. The solution was put into the chromatographic column and after division into fractions IgG immunoglobulins were eluated 0,025 M tris-HCE pH 7,8-0,15 M tris - HCE with pH 7,8 buffer. The solution was dialyzed through the permselective membrane against distilled water within 2 days. After the dyalysis, the immunoglobulins solution was sterilized by filtering through Millipore's filters 0,22 mkm. The liquid sterile solution was put into ampullas per 1 ml. The protein content in a dose was 5 mg. Desiccation by lyophilizing was held in a regime of immunoglobulins lyophilization (from -60°C till 37°C). The ampullas were hermetically sealed up. The agent was being kept till application at a temperature of 4°C.

The antigen titer in the prepared agent constituted 1:1200 to CD34(-) mesenchymal cells antigen. A residual moisture 3% mass. The lyophilized agent represent a white porous hygroscopic mass completely dissoluble in water or physiological solution at indoor temperature within 60 sec. The ampullas content dissolves in 1 ml water, the solution is transparent and slightly opalescent.

The agent was under investigation for sterility, toxicity and apyrogenicity in accordance with regulated techniques. Specific innocence was being defined by its capability to cause hemagglutination and thromboagglutination in respective tests and lymphotoxicity in microlymphocytotoxicity assay by Terasaky as well [Ketlinskii S.A., Kalinina N.M. Immunology for doctor. Saint-Petersburg, 1998, 156 p], while the claimed agent concentrates action on blood corpuscle.

The agent did not possess toxic property in delutions under test.

Morphological investigations under light and electronic microscopy did not reveal toxic characteristics - vacuolization and structural rupture of cytoplasm, its colouring by standard dyes, - cells organelle integrity not defected.

The specific activity of the obtained agent was defined as follows.

There was held a test in vitro to investigate the claimed agent effects for growth of mesenchymal cells and their descendants.

The mesenchymal cells and their descendants were extracted out of fragments of human trabecular bone taken during the trepanobiopsy. The fragments of trabecular bone were put into nutritional medium in Petri dishes to the gas-running incubator at a temperature of 36,8±0,2°C. The claimed agent was added in test dishes in a dose 0,0125 mkg/ml nutritional medium. Endurance in incubator was 2 weeks. A number of cellular aggregates (3 and more cells) was measured microscopically on cover glass of square 567 mm. The result was compared to samples in which the claimed agent was not introduced (endurance and conditions are identical).

### The investigation results represented in table.

**Table**

| Number of cellular aggregates per 1 sample of bioptate | | | | | | | |
|---|---|---|---|---|---|---|---|
| Normoplastic type | | | | Hypoplastic type | | | |
| Checking | | Testing | | Checking | | Testing | |
| Number of aggregates | Number of cells in aggregates | Number of aggregates | Number of cells in aggregates | Number of aggregates | Number of cells in aggregates | Number of aggregates | Number of cells in aggregates |
| 5 - 9 | 20-45 | 10-12 | 30-50 | 2-4 | 6-12 | 5-7 | 15-21 |

According to the table, the claimed agent injection brings to increasing aggregates up to the norm while the hypoplastic type and increases 30% and more in comparison with the initial state while the normoplastic type.

There was held a test in vivo to demonstrate the claimed agent activity. To obtain antigens there were taken fragments of Vistar's rats tubular bones that contains mesenchymal cells.

According to the table, the claimed agent injection brings to increasing aggregates up to the norm while the hypoplastic type and increases 30% and more in comparison with the initial state while the normoplastic type.

There was held a test in vivo to demonstrate the claimed agent activity. To obtain antigens there were taken fragments of Vistar's rats tubular bones that contains mesenchymal cells.

The bioptate was treated the identical way as described above dealing with human material. To obtain immunoglobulins a 2,2 kg rabbit was immunized 6 times, using 4,4 mg antigen for each injection. Before the sixth injection the antibodies titer was 1:1024. The agent was being obtained as described above.

The immunized rabbit blood was taken on the 7-th day after the sixth injection. After the blood clot retraction the serum was taken with dropping pipettes and centrifugated at a speed of 2500 r/min during 20 min. The agent was being obtained out of the extracted serum as described above.

8 Vistar's rats were subjected to the mechanical fracture of lower leg bone. Treatment started in the same day. 4 rats of the test group took 5 intramuscular injections of the immune agent everyday; 2 rats did not take any injections; 2 rats took 5 injections of non-immune agent. The agent was dissolved in 1,0 ml water and injected counting 0,1 ml/kg to animal body. On the 7-th day after the first injection were decapitated under ether raush-narcosis 2 rats from the test group, 1 rat from «pure control» group and 1 rat from the treatment control group. Agents of lower leg fracture areas were investigated histologically.

On the 7-th day in the control groups there was revealed formation of fibrous tissue 2-4 times more than cartilage dimensions. The blood vessels grew in the regeneration areas but their entire space did not exceed 6% within of eyeshot.

On the 7-th day in the test group there was registered a distinct domination of cartilaginous tissue over fibrous tissue and primordiums of osteogenic tissue appeared over fibroreticular tissue areas. The space of forming vascular tissue was 23% in the regeneration areas.

On the 14-th day in the control group there was registered formation of the osteotylus on which periphery the fibrous tissue and osteogenic tissue were dominating. The size of the osteogenic tissue was growing and became equal to the size of the fibrous tissue. The space of the forming vessels increased up to 9%. There had been formed single trabeculas of bone in the periosteum area near which there was found formation of endothelium.

On the 14-th day in the test group the animals had osteotylus formed by osseous tissue and cartilage. The trabeculas of bone were formed along the whole osteotylus length. Because of the osseous tissue formation the space of terebrant vessels got less down to the norm 14,5%. In the vessels in growth the endotheliocytes were characterized with a higher functional activity, that is, existence of abundant cytoplasm containing large-size nuclcuses. The tissue degeneration signs or abnormal cellular forms were not revealed.

Thus, the serum application for the treatment of tubular bones mechanical fracture during the test resulted in full formation of osteotylus within earlier periods.

### Feasibility.

The claimed agent may be used to treat diseases that cause cellular and tissues structure rupture and require their regeneration. The agent may be produced by biotechnological branch of pharmaceutical industry.

## Claims

1. Agent for actively restoring structure and functions of damaged tissues and organs differs because contains as immunoglobulins IgG-class polyclonals of average molecular mass 150 kD obtained as humoral immune response to the antigen complex of CD34(-) mesenchymal cells differs because contains as immunoglobulins IgG-class polyclonals of average molecular mass 150kD obtained as humoral immune response to the antigen complex of CD34(-) mesenchymal cells.

2. Agent for actively restoring structure and functions of damaged tissues and organs according to paragraph 1 differs because contains xenogenous IgG-class polyclonals to the antigen complex of CD34(-) mesenchymal cells.
